# EUROPÄISCHE PATENTSCHRIFT

(11) **EP 2 677 981 B1**
(45) Veröffentlichungstag und Bekanntmachung des Hinweises auf die Patenterteilung: **01.04.2020**
(21) Anmeldenummer: 11708217.2
(22) Anmeldetag: 23.02.2011
(51) Int. Cl.: A61F 9/007

(54) **IMPLANTAT ZUR BEHANDLUNG VON GLAUKOM**
IMPLANT FOR TREATING GLAUCOMA
IMPLANT POUR LE TRAITEMENT DU GLAUCOME

(43) Veröffentlichungstag der Anmeldung: 01.01.2014
(73) Patentinhaber: Grieshaber Ophthalmic Research Foundation, 9000 St. Gallen (CH)
(72) Erfinder: GRIESHABER, Hans R., 8200 Schaffhausen (CH); GRIESHABER, Matthias, 4102 Binningen (CH); STEGMANN, Robert, 0081 Pretoria (ZA)
(74) Vertreter: Dr. Graf & Partner AG
(86) Internationale Anmeldenummer: PCT/EP2011/052683
(87) Internationale Veröffentlichungsnummer: WO 2012/113450

(56) Entgegenhaltungen:
- WO-A1-2008/002377
- WO-A1-2010/072574
- WO-A2-2004/026347
- WO-A2-2009/042596
- US-A1- 2007 088 432
- US-A1- 2008 228 127

## Beschreibung

Die Erfindung bezieht sich auf ein Implantat zur Behandlung von Glaukom, bestehend aus einem in den an mindestens einer Stelle der Sklera freigelegten Schlemmschen Kanal einführbaren Röhrchen, welches flexibel biegsam ausgebildet ist und für den trans-trabelukulären Kammerwasser-Abfluss mehrere in der Wand in axialer Richtung sowie in Umfangsrichtung im Abstand zueinander angeordnete und mit dem Innenraum des Röhrchens in Verbindung stehende erste Ausnehmungen aufweist.

### Ophthalmologischer Hintergrund

Bei einem gesunden Auge erfolgt der Abfluss des zirkulierenden Kammerwassers (Humor aquosus) von der Hinterkammer zur Vorderkammer und wird im Kammerwinkel (Angulus iridocornealis) über das Trabekulargewebe in den Schlemmschen Kanal und von dort über das episklerale Venensystem in den Blutkreislauf abgeleitet. Bei krankhaften Zuständen des Auges, insbesondere bei auftretenden Widerständen aufgrund eines beispielsweise durch Verklebung oder dergleichen geschlossenen Schlemmschen Kanals ist ein kontinuierlicher Abfluss des vom Epithel des Ziliarkörpers gebildeten und sich ständig erneuernden Kammerwassers nicht ausreichend oder gar nicht mehr gewährleistet. Hierdurch kann der Augeninnendruck (IOP) derart ansteigen, dass die Durchblutung der Sehnerven und somit die Funktion derselben eingeschränkt wird. Diese Funktionstörung kann zu der als Glaukom oder grüner Star bekannten Augenkrankheit beziehungsweise bis zum Erblinden des jeweiligen Auges oder beider Augen führen.

### Stand der Technik

Aus der Druckschrift (WO 2010/072574 A1) ist ein aus einem länglichen flexiblen Röhrchen hergestelltes und in den Schlemmschen Kanal des Auges einführbares Implantat bekannt, welches mehrere in axialer Richtung beabstandete Ringteile, mehrere zwischen den Ringteilen angeordnete und mit dem Innenraum des Röhrchens in Verbindung stehende Ausnehmungen sowie mindestens ein in axialer Richtung orientiertes Verbindungsteil umfasst, welches derart mit einer kreisbogenförmigen Oberfläche an der Innenwand des Schlemmschen Kanals anliegt, dass die einzelnen Ausnehmungen für den Abfluss des Kammerwassers eine direkte Verbindung von dem Trabekulargewebe zu dem episkleralen Venensystem bilden.

Zur Behandlung von Glaukom sind aus der Druckschrift (EP 0 898 947 A3) unterschiedlich ausgebildete Stützelemente bekannt, welche beispielsweise als längliches Röhrchen mit verteilt angeordneten Löchern oder als längliches Stützelement beziehungsweise als röhrchenförmiges Netzgeflecht ausgebildet sind und in den durch einen Einschnitt und aufgeklappten Skleralappen freigelegten und von einem eingespritzten hochviskosen Medium mechanisch gedehnten Schlemmschen Kanal einführbar und am Zielort freisetzbar sind. Mittels der Stützelemente wird der natürliche Abfluss des zirkulierenden und sich permanent erneuernden Kammerwassers von der Vorderkammer über das Trabekulargewebe in den zirkulären Schlemmschen Kanal und von dort über das episklerale Venensystem in den Blutkreislauf verbessert.

Aus der Druckschrift (US 2004/0210181 A1) ist weiterhin ein mittels einer Platte an der mit einem Einschnitt (Inzision) versehenen Sklera fixierbares und im Profilquerschnitt etwa T-förmig ausgebildetes Implantat bekannt, welches ein operativ in die Vorderkammer oder durch das Trabekulargewebe in die Vorderkammer einführbares proximales Rohrstück sowie zwei gegenüberliegend zueinander an dem Rohrstück angeordnete und in den freigelegten Schlemmschen Kanal einführbare distale Röhrchen umfasst. Mit dem im wesentlichen als Drainage ausgebildeten Implantat wird bei krankhaft verschlossenem Trabekulargewebe das sich permanent erneuernde Kammerwasser zur Vermeidung eines erhöhten Innendrucks (IOP) auf künstlichem Weg von dem in die Vorderkammer eingeführten proximalen Rohrstück über die beiden mit Ausnehmungen versehenen distalen Röhrchen in den Schlemmschen Kanal und von dort über das episklerale Venensystem in den Blutkreislauf des Auges abgeleitet.

### Darstellung der Erfindung

Der Erfindung liegt die Aufgabe zugrunde ein in den Schlemmschen Kanal einführbares und als Implantat ausgebildetes Röhrchen anzugeben, mittels welchem eine den Augeninnendruck regulierende Zirkulation des Kammerwassers über das Lumen des zirkulären Schlemmschen Kanals erreicht wird und somit der natürliche trans-trabekuläre Abfluss des Kammerwassers in das episklerale Venensystem und in den Blutkreislauf des Auges verbessert und permanent aufrechterhalten wird.

Das erfindungsgemässe Implantat ist dadurch gekennzeichnet, dass die ersten Ausnehmungen als geometrisches Muster verteilt zueinander in der Wand des Röhrchens angeordnet sind und das Röhrchen ein im Profilquerschnitt entsprechend kreisbogenförmig ausgebildetes und in axialer Richtung orientiertes Segmentteil mit in axialer Richtung im Abstand zueinander angeordneten zweiten Ausnehmungen aufweist, welche analog wie die ersten Ausnehmungen mit dem Innenraum des Röhrchens in Verbindung stehen.

Eine bevorzugte Ausführungsform des Implantats wird darin gesehen, dass bei der Laserbearbeitung an der Oberfläche sowie an den Kanten entstandene Grate, Auffaserungen oder dergleichen beispielsweise durch eine thermische Entgratung entfernbar sind und zudem bei der Laserbearbeitung entstandenen Kanten mit geeigneten Mitteln abgerundet sind.

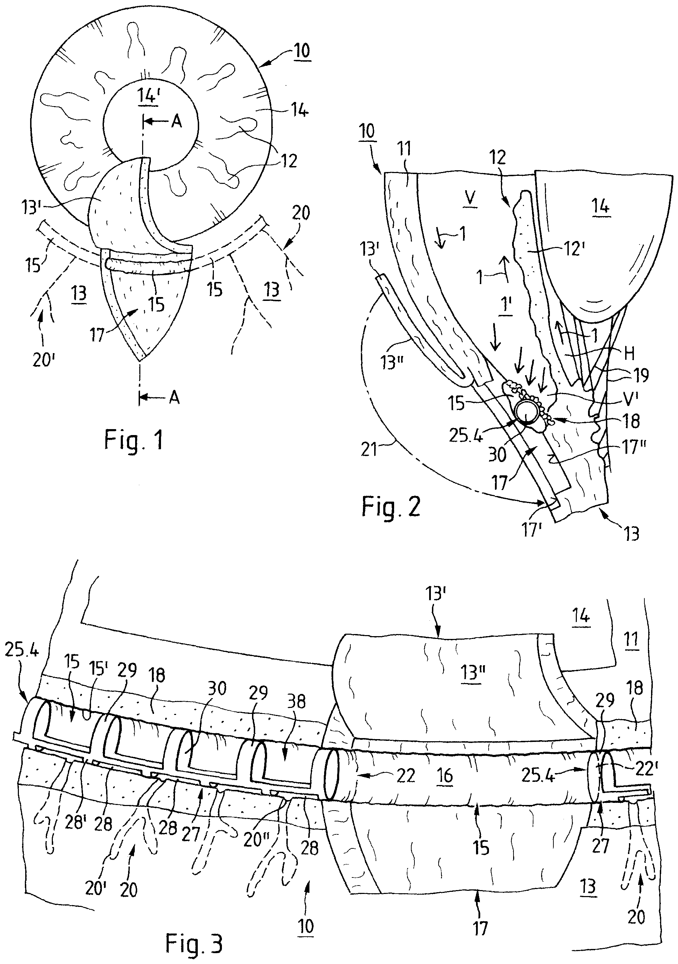

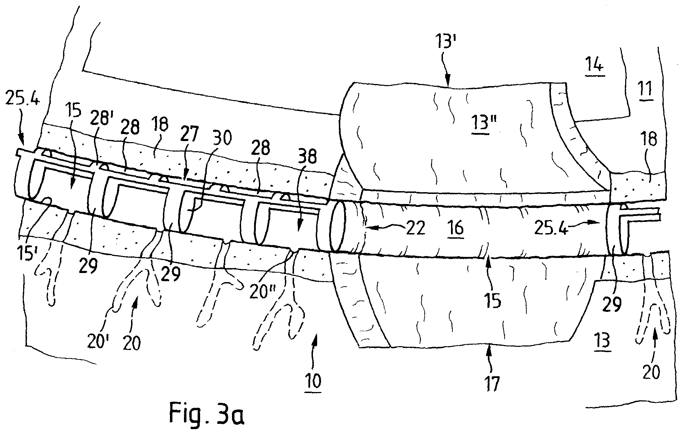

Das gemäss Fig.3 in das Lumen des Schlemmschen Kanals eingeführte Röhrchen hat den Vorteil, dass der Schlemmsche Kanal einerseits permanent offen gehalten und stabilisiert wird und andererseits der natürliche trans-trabekuläre Abfluss des Kammerwassers von dem Trabekulargewebe durch die ersten Ausnehmungen in den Innenraum des Implantats und von dort durch die zweiten Ausnehmungen in die Öffnungen der einzelnen natürlich verteilten Kanälchen des episkleralen Venensystems und von dort in den Blutkreislauf abgeleitet wird.

Bei der in Fig.3a dargestellten Variante ist das Röhrchen um seine Längsachse etwa um 180° gedreht in den Schlemmschen Kanal eingeführt, so dass der natürliche trans-trabekuläre Abfluss des Kammerwassers von dem Trabekulargewebe zunächst durch die im Segmentteil vorgesehenen zweiten Ausnehmungen in den Innenraum des Implantats und von dort durch die zwischen den Ringteilen vorgesehenen ersten Ausnehmungen in die damit in Verbindung stehenden Öffnungen der einzelnen natürlichen Kanälchen des episkleralen Venensystems und von dort in den Blutkreislauf abgeleitet wird.

### Beschreibung der Zeichnungen

Das als Implantat ausgebildete längliche Röhrchen und Einzelheiten desselben sowie zweckmässige weitere Ausgestaltungen werden nachstehend anhand der Zeichnung beschrieben, wobei die Ausführungsformen gemäss der Figuren 4 bis 7 nicht Teil der Erfindung sind. Es zeigt:
- **Fig. 1**: ein in schematischer Ansicht dargestelltes Auge mit dem durch einen lamellaren Einschnitt und aufgeklapptem Skleralappen freigelegten Schlemmschen Kanal zum Einführen eines als Implantat ausgebildeten Röhrchens;
- **Fig. 2**: ein gemäss der in Fig.1 eingezeichneten Schnittlinie A-A in grösserem Massstab dargestelltes Teilstück des Auges mit dem im Schlemmschen Kanal angeordneten Implantat;
- **Fig. 3**: ein in grösserem Massstab und im Schnitt dargestelltes Teilstück des Auges mit dem gemäss einer ersten Variante in den freigelegten Schlemmschen Kanal eingeführten Implantat;
- **Fig.3a**: das im Schnitt dargestellte Teilstück des Auges gemäss Fig.3 mit dem gemäss einer zweiten Variante in den freigelegten Schlemmschen Kanal eingeführten Implantat;
- **Fig. 4**: einen in räumlicher Ansicht dargestellten röhrchenförmigen Rohling für das in der Wand gemäss einem geometrischen Muster mit verteilt zueinander angeordneten Ausnehmungen auszubildende Implantat;
- **Fig. 5**: die als Abwicklung und in Draufsicht dargestellte Wand des Röhrchens mit entsprechend einem ersten geometrischen Muster verteilt angeordneten ersten Ausnehmungen;
- **Fig. 5a**: ein in grösserem Massstab und in Draufsicht dargestelltes Teilstück der Wand gemäss Fig.5 mit den gemäss dem ersten geometrischen Muster verteilt angeordneten ersten Ausnehmungen;
- **Fig. 6**: ein als Abwicklung und in Draufsicht dargestelltes Teilstück des Röhrchens mit den nach einem zweiten geometrischen Muster in der Wand angeordneten ersten Ausnehmungen;
- **Fig. 7**: ein als Abwicklung und in Draufsicht dargestelltes Teilstück des Röhrchens mit den nach einem dritten geometrischen Muster in der Wand angeordneten ersten Ausnehmungen;
- **Fig. 8**: ein in räumlicher Ansicht dargestelltes Röhrchen mit den nach einem vierten geometrischen Muster verteilt zwischen einzelnen Ringteilen angeordneten ersten Ausnehmungen;
- **Fig. 8a**: ein als Abwicklung und in Draufsicht dargestelltes Teilstück des Röhrchens gemäss Fig.8 mit den verteilt zwischen den Ringteilen angeordneten ersten Ausnehmungen;
- **Fig. 9**: das als Abwicklung und in Draufsicht dargestellte Teilstück des Röhrchens gemäss Fig.8a mit den nach einem weiteren geometrischen Muster verteilt zwischen den Ringteilen angeordneten ersten Ausnehmungen; und
- **Fig. 10**: das in Ansicht sowie im Profilquerschnitt dargestellte Röhrchen gemäss Fig.8.

### Figurenbeschreibung

Fig.1 bis 3 zeigen zum besseren Verständnis der Problematik in Verbindung mit der Glaukomchirurgie jeweils ein Teilstück des Auges, wobei in Fig.2 und in Fig.3 jeweils ein mit nicht näher dargestellten Mitteln in den Schlemmschen Kanal eingeführtes und als längliches Röhrchen ausgebildetes Implantat dargestellt ist.

Fig.1 zeigt in schematischer Frontansicht ein in der Gesamtheit mit 10 bezeichnetes Auge und man erkennt die Linse 14 mit der Pupille 14', die Regenbogenhaut 12, die Lederhaut 13, den teilweise dargestellten Schlemmschen Kanal 15 sowie die damit in Verbindung stehenden Kammerwasservenen 20 (collector channels) mit den einzelnen Kanälchen 20'.

Mit einem mikrochirurgischen Eingriff wird in an sich bekannter Weise, wie in Fig.1 schematisch dargestellt, die Lederhaut 13 lamellar eingeschnitten und nach dem Abtrennen eines nicht näher dargestellten Teilstücks das äussere Teilstück 13' aufgeklappt und mit nicht dargestellten Mitteln gehalten. Der lamellare Einschnitt bildet im Bereich des freigelegten Schlemmschen Kanals 15 ein mit 17 bezeichnetes Sklerabett, welches nach dem Einführen und Freisetzen des als Röhrchen ausgebildeten Implantats wieder verschlossen wird.

Fig.2 zeigt das gemäss der in Fig.1 eingezeichneten Linie A-A im Schnitt sowie in grösserem Massstab dargestellte Teilstück des Auges 10 und man erkennt die Hornhaut 11, das eine Teilstück 12' der Regenbogenhaut 12, die Lederhaut 13 mit dem aufgeklappten Skleralappen 13', die Linse 14, die Zonulafasern 19, die Hinterkammer H und die Vorderkammer V mit dem Kammerwinkel V' sowie das dem Schlemmschen Kanal 15 vorgelagerte Trabekulargewebe 18. Der zirkulär um die Linse 14 orientierte Schlemmsche Kanal 15 ist, wie in Fig.2 schematisch dargestellt, im Profilquerschnitt etwa als längliches Oval ausgebildet, welches ausgehend von dem einen Ende im Bereich des Kammerwinkels V' in Richtung des anderen gegenüberliegenden Endes eine verjüngende Formgebung haben kann.

Weiterhin erkennt man in Fig.2 das in den Schlemmschen Kanal 15 eingeführte Röhrchen sowie das Sklerabett 17 mit der Innenfläche 17" und der Auflagefläche 17' für den in Pfeilrichtung 21 herunterklappbaren Skleralappen 13', welcher mit der Innenseite 13" an der Auflagefläche 17' des Sklerabetts 17 aufliegt und mit nicht dargestellten Mitteln befestigt wird. In Fig.2 ist als Ausführungsbeispiel das als Implantat ausgebildete Röhrchen 25.4 mit dem Innenraum 30 dargestellt. Weiterhin ist in Fig.2 die von der Hinterkammer H in Richtung der Vorderkammer V orientierte und mit den Pfeilen 1 bezeichnete Zirkulation des Kammerwassers dargestellt. Das Kammerwasser gelangt gemäss der Pfeile 1' auf natürlichem Weg von dem Kammerwinkel V' durch das Trabekulargewebe 18 in den Schlemmschen Kanal 15 und von dort über das episklerale Venensystem in den Blutkreislauf.

Fig.3 und Fig.3a zeigen jeweils ein in grösserem Massstab und teilweise im Schnitt dargestelltes Teilstück des Auges 10 mit dem Sklerabett 17 und dem aufgeklappten Skleralappen 13' sowie das beispielsweise durch die freigelegte erste Öffnung 22 in das Lumen 16 des zirkulären Schlemmschen Kanals 15 eingeführte Röhrchen 25.4.

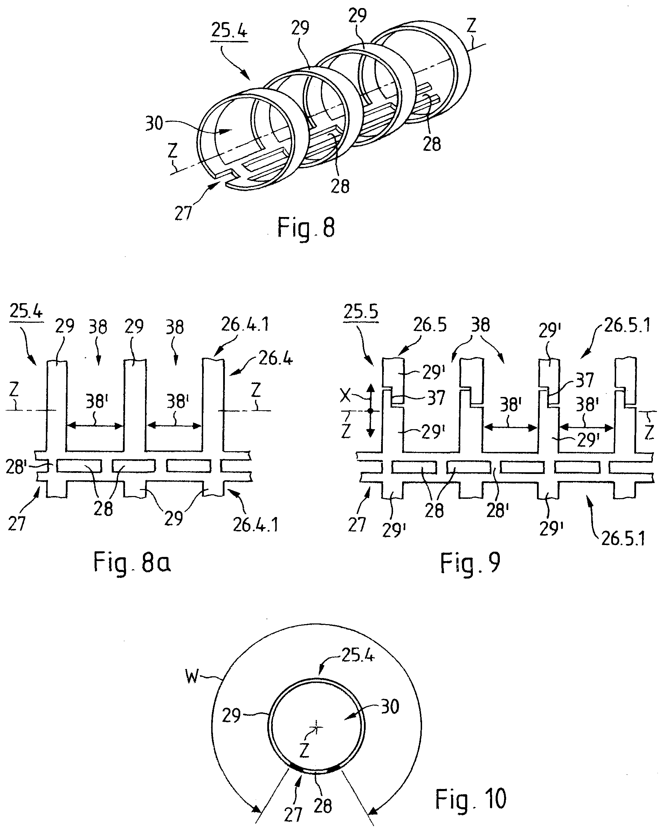

Das später in Verbindung mit Fig.8 näher beschriebene und als Implantat ausgebildete Röhrchen 25.4 hat mehrere in axialer Richtung im Abstand zueinander angeordnete und im wesentlichen kreisbogenförmig ausgebildete und an einem in axialer Richtung orientierten Segmentteil 27 angeordnete Ringteile 29.

Das in den Schlemmschen Kanal 15 eingeführte und flexibel ausgebildete Implantat hat, wie in Fig.3 und Fig.3a dargestellt, beispielsweise eine in Umfangsrichtung des Schlemmschen Kanals 15 von der ersten Öffnung 22 bis zu der gegenüberliegenden zweiten Öffnung 22' reichende Länge und ist entsprechend der natürlichen Formgebung des zirkulären Schlemmschen Kanals 15 selbsttätig anpassbar.

Bei der in Fig.3 dargestellten ersten Variante ist das Röhrchen 25.4 derart in den Schlemmschen Kanal 15 eingeführt und darin angeordnet, dass die einzelnen Ringteile 29 dem Trabekulargewebe 18 zugewandt und an der Innenwand 15' des Schlemmschen Kanals 15 anliegen, wobei bei dieser Anordnung (Fig.3) das Segmentteil 27 mit darin angeordneten Ausnehmungen 28 mit den Kanälchen 20' und Öffnungen 20" der schematisch dargestellten Kammerwasservenen 20 (collector channels) zugeordnet sind und damit in Verbindung stehen.

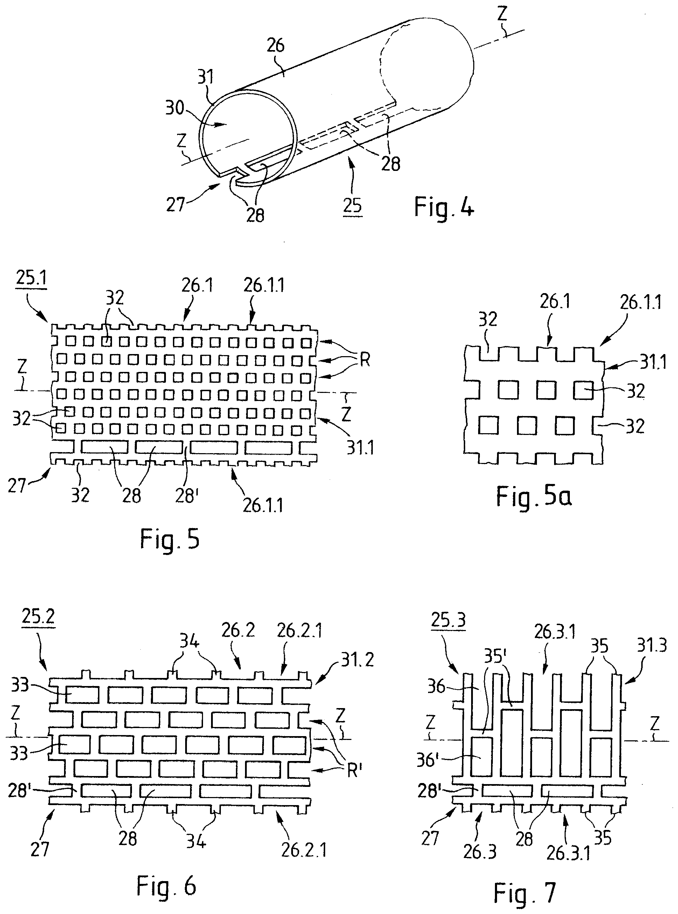

Bei der in Fig.3a dargestellten zweiten Variante ist das Röhrchen 25.4 um die Längsachse Z (Fig.4 und 8) etwa um 180° gedreht in den Schlemmschen Kanal 15 eingeführt und derart darin angeordnet, dass die einzelnen im Abstand zueinander angeordneten Ringteile 29 der mit den Kanälchen 20' der Kammerwasservenen 20 in Verbindung stehenden Innenwand 15' des Schlemmschen Kanals 15 und mit dem in axialer Richtung des Röhrchens 25.4 orientierten Segmentteil 27 der gegenüberliegenden und mit dem Trabekulargewebe 18 in Verbindung stehenden Innenwand 15' des Schlemmschen Kanals 15 zugeordnet sind.

An dieser Stelle wird darauf hingewiesen, dass das als Implantat ausgebildete Röhrchen 25.4 beispielsweise in Abhängigkeit der organischen und anatomischen Beschaffenheit des zirkulären Schlemmschen Kanals 15 in das Lumen 16 desselben eingeführt wird und infolge davon der natürliche trans-trabekuläre Abfluss des Kammerwassers in das episklerale Venensystem und in den Blutkreislauf des Auges verbessert und permanent aufrechterhalten wird.

Zur Optimierung des trans-trabekulären Kammerwasser-Abflusses kann der Schlemmsche Kanal durch eine zirkumferenzielle Dilatation mechanisch gedehnt und anschliessend das erfindungsgemässe Implantat (Röhrchen) in das gedehnte Lumen eingeführt und beispielsweise am Zielort freigesetzt werden.

In Fig.4 ist zur Erläuterung unterschiedlich ausgebildeter Implantate ein röhrchenförmiger Rohling 25 in räumlicher Ansicht dargestellt. Der Rohling 25 hat einen mit einer Wand 31 versehenen hohlzylindrischen Mantel 26 mit einem Innenraum 30, ein in axialer Richtung orientiertes Segmentteil 27 sowie eine den Innenraum 30 in axialer Richtung durchdringende Längsachse Z. Das in Längsrichtung orientierte Segmentteil 27 ist im Profilquerschnitt entsprechend dem Rohling 25 kreisbogenförmig ausgebildet und hat mehrere in axialer Richtung im Abstand zueinander angeordnete Ausnehmungen 28.

Nachstehend werden einzelne aus dem röhrchenförmigen Rohling 25 hergestellte und mit ersten Ausnehmungen 32,33,36,36'und 38 sowie mit mehreren zweiten Ausnehmungen 28 versehene und als Implantat ausgebildete Röhrchen 25.1 bis 25.5 beschrieben. Die ersten Ausnehmungen 32,33,36,36' und 38 sind entsprechend einem geometrischen Muster beziehungsweise einer geometrischen Struktur in der Wand 31 des Röhrchens 25.1 bis 25.5 angeordnet. Die zwischen den Stegen 28' angeordneten und in Richtung der Längsachse Z orientierten zweiten Ausnehmungen 28 sind derart in dem in axialer Richtung orientierten Segmentteil 27 des Röhrchens 25.1 bis 25.5 im Abstand zueinander angeordnet, dass die einzelnen Öffnungen 20" der Kammerwasservenen 20 (collector channels) für das Kammerwasser frei zugänglich sind (vgl. Fig.3 und Fig.3a). Die zwischen den einzelnen Stegen 28' angeordneten und beispielsweise als Langloch ausgebildeten zweiten Ausnehmungen 28 sind gleich gross oder unterschiedlich gross ausgebildet.

Fig.5 zeigt ein erstes Ausführungsbeispiel des Röhrchens 25.1 mit dem in Draufsicht und als Abwicklung dargestellten Mantel 26.1. Der mit den nach einem ersten geometrischen Muster verteilt angeordneten ersten Ausnehmungen 32 versehene Mantel 26.1 umfasst das parallel zu der Längsachse Z in axialer Richtung orientierte Segmentteil 27 mit den Stegen 28' und den in axialer Richtung im Abstand zueinander angeordneten zweiten Ausnehmungen 28. Der im dargestellten Ausführungsbeispiel mit den versetzt zueinander in der Wand 31.1 in Reihen R angeordneten ersten Ausnehmungen 32 versehene Mantel 26.1 ist in Fig.5 in aufgeklapptem Zustand dargestellt. Zu beiden Seiten des in axialer Richtung orientierten Segmentteils 27 ist jeweils ein Teilstück 26.1.1 des Mantels 26.1 dargestellt. Die beiden Teilsstücke 26.1.1 bilden in zusammengefügtem Zustand das als Implantat ausgebildete Röhrchen 25.1 mit einer beispielsweise siebförmig gelochten ersten Mantelstruktur.

In Fig.5a ist in grösserem Massstab das Teilstück 26.1.1 des Mantels 26.1 dargestellt und man erkennt die in der Wand 31.1 im Abstand zueinander angeordneten ersten Ausnehmungen 32, welche in versetzt zueinander angeordneten und in Richtung der Längsachse Z orientierten Reihen R sowie quer dazu im Abstand zueinander angeordnet sind. Die ersten Ausnehmungen 32 können, wie in Fig.5 und Fig.5a dargestellt, quadratisch oder in nicht näher dargestellter Weise kreisförmig oder oval ausgebildet werden.

Fig.6 zeigt ein zweites Ausführungsbeispiel des Röhrchens 25.2 mit dem in Draufsicht und als Abwicklung dargestellten Mantel 26.2. Der mit einem zweiten geometrischen Muster mit verteilt angeordneten und beispielsweise als rechteckiges Langloch ausgebildeten ersten Ausnehmungen 33 versehene Mantel 26.2 umfasst das in Richtung der Längsachse Z orientierte Segmentteil 27 mit den in axialer Richtung im Abstand zueinander angeordneten zweiten Ausnehmungen 28 und den dazwischen angeordneten Stegen 28'. In aufgeklapptem Zustand ist zu beiden Seiten des Segmentteils 27 jeweils ein mit Stegen 34 versehenes Teilstück 26.2.1 des Mantels 26.2 dargestellt. In zusammengefügtem Zustand bilden die beiden Teilstücke 26.2.1 das als Implantat ausgebildete Röhrchen 25.2 mit der gelochten Mantelstruktur. Der Mantel 26.2 umfasst eine Vielzahl in der Wand 31.2 im Abstand zueinander angeordnete erste Ausnehmungen 33, welche in Richtung der Längsachse Z in versetzten und im Abstand zueinander orientierten Reihen R' angeordnet sind. Bei dieser Variante sind die ersten Ausnehmungen 33 beispielsweise eckig oder oval ausgebildet und von der einen Reihe R' in Bezug auf die andere Reihe R' vesetzt zueinander angeordnet. Die beiden Teilsstücke 26.2.1 bilden in zusammengefügtem Zustand das als Implantat ausgebildete Röhrchen 25.2 mit einer langlochförmig gelocht ausgebildeten zweiten Mantelstruktur.

Fig.7 zeigt ein drittes Ausführungsbeispiel des Röhrchens 25.3 mit dem in Draufsicht und als Abwicklung dargestellten Mantel 26.3. Der mit einem dritten geometrischen Muster mit etwa gitterförmig in der Wand 31.3 verteilt angeordneten ersten Ausnehmungen 36 und 36' versehene Mantel 26.3 umfasst das parallel zu der Längsachse Z in axialer Richtung orientierte Segmentteil 27 mit den in axialer Richtung im Abstand zueinander angeordneten zweiten Ausnehmungen 28 sowie die dazwischen angeordneten Stege 28'. In aufgeklapptem Zustand ist zu beiden Seiten des Segmentteils 27 jeweils ein mit radial orientierten ersten Stegen 35 und quer dazu angeordneten zweiten Stegen 35' versehenes Teilstück 26.3.1 des Mantels 26.3 dargestellt. Zwischen den einzelnen in Umfangsrichtung orientierten ersten Stegen 35 und den dazwischen in axialer Richtung angeordneten zweiten Stege 35' sind die ersten Ausnehmungen 36 und 36' versetzt sowie im Abstand zueinander angeordnet. In zusammengefügtem Zustand bilden die beiden Teilstücke 26.3.1 das als Implantat ausgebildete Röhrchen 25.3 mit der im wesentlichen gitterförmig (grid-like) ausgebildeten dritten Mantelstruktur.

In Fig.8 ist als viertes Ausführungsbeispiel das Röhrchen 25.4 in räumlicher Ansicht dargestellt und man erkennt das in Richtung der Längsachse Z orientierte Segmentteil 27 mit den im Abstand zueinander angeordneten zweiten Ausnehmungen 28 sowie mehrere im Abstand zueinander an dem Segmentteil 27 angeordnete Ringteile 29. Die im wesentlichen den Innenraum 30 des Röhrchens 25.4 bildenden Ringteile 29 sind vorzugsweise an dem in axialer Richtung des Röhrchens 25.4 orientierten Segmentteil 27 in nicht näher dargestellter Weise angeformt.

Fig.8a zeigt das Röhrchen 25.4 gemäss Fig.8 mit dem in Draufsicht und als Abwicklung dargestellten Mantel 26.4 und man erkennt das in axialer Richtung orientierte Segmentteil 27 mit den einzelnen im Abstand zueinander angeordneten zweiten Ausnehmungen 28 sowie die dazwischen angeordneten Stege 28'. Die bei diesem Ausführungsbeispiel in Umfangsrichtung des Röhrchens 25.4 orientierten ersten Ausnehmungen 38 sind jeweils mit dem Abstand 38' zwischen den einzelnen Ringteilen 29 angeordnet. Der Abstand 38' beträgt beispielsweise das zwei- bis dreifache der Breite des einzelnen Ringteils 29. In zusammengefügtem Zustand bilden die beiden mit dem Segmentteil 27, den Ringteilen 29 und mit den ersten und zweiten Ausnehmungen versehenen Teilstücke 26.4.1 das als Implantat ausgebildete Röhrchen 25.4. Die einzelnen parallel zueinander angeordneten Ringteile 29 bilden eine weitgehend gitterförmige (grid-like) vierte Mantelstruktur.

In Fig.9 ist ein weiteres Ausführungsbeispiel des Röhrchens 25.5 mit dem Mantel 26.5 in Draufsicht und als Abwicklung dargestellt und man erkennt das in axialer Richtung orientierte Segmentteil 27 mit den einzelnen im Abstand zueinander angeordneten zweiten Ausnehmungen 28 mit den Stegen 28' sowie die mit den ersten Ausnehmungen 38 in axialer Richtung im Abstand 38' zueinander angeordneten und an dem Segmentteil 27 angeformten Ringteile 29. In zusammengefügtem Zustand bilden die beiden Teilstücke 26.5.1 das als Implantat ausgebildete Röhrchen 25.5 mit der im wesentlichen gemäss Fig.8a analog gitterförmig ausgebildeten vierten Mantelstruktur.

Bei der Variante gemäss Fig.9 sind die in axialer Richtung im Abstand zueinander angeordneten Ringteile 29 am äusseren Umfang jeweils mit einem etwa z-förmig ausgebildeten Schlitz oder Spalt 37 in zwei halbkreisförmige Teilstücke 29' unterteilt. Die beiden Teilstücke 29' sind jeweils in Bezug auf die Längsachse Z gemäss Pfeilrichtung X in radialer Richtung beispielsweise zum Freisetzen in dem Schlemmschen Kanal 15 relativ zueinander spreizbar. Der bei diesem Ausführungsbeispiel die ersten Ausnehmungen 38 bildende Abstand 38' zwischen den einzelnen Ringteilen 29 beträgt etwa das zwei- bis dreifache der Breite des einzelnen Ringteils 29.

In Fig.10 ist das mit den Ringteilen 29 und dem Innenraum 30 versehene Röhrchen 25.4 beziehungsweise 25.5 im Profilquerschnitt dargestellt und man erkennt das kreisförmige Ringteil 29 sowie das in axialer Richtung orientierte und mit der zweiten Ausnehmung 28 versehene kreisbogenförmig ausgebildete Segmentteil 27. Die zwischen den einzelnen Ringteilen 29 angeordneten ersten Ausnehmungen 38 haben jeweils einen in der Grössenordnung zwischen 290° bis 310° liegenden Öffnungswinkel W.

Die in den Figuren 5 bis 9 als Langloch ausgebildeten und in dem Segmentteil 27 angeordneten zweiten Ausnehmungen 28 sowie die in dem Röhrchen 25.2 (Fig.6) angeordneten ersten Ausnehmungen 33 sind jeweils als rechteckiges Langloch ausgebildet. Bei einer nicht näher dargestellten Variante besteht die Möglichkeit, dass das einzelne Langloch an den gegenüberliegenden Enden jeweils mit einer halbkreisförmig ausgebildeten Stirnseite versehen ist.

Die vorstehend in Verbindung mit den Figuren 4 bis 10 beschriebenen und jeweils als Implantat ausgebildete Röhrchen 25.1 bis 25.5 haben einen äusseren Durchmesser in der Grössenordnung von etwa 0,15 mm bis 0,35 mm sowie einen inneren, lichten Durchmesser in der Grössenordnung von 0,1 mm bis 0,25 mm. Die in der Mantelwand des Röhrchens 25.1 bis 25.5 angeordneten und jeweils als geometrisches Muster oder als geometrische Struktur ausgebildeten ersten Ausnehmungen 32,33,36,36' und 38 sowie die in dem in axialer Richtung orientierten Segmentteil 27 angeordneten zweiten Ausnehmungen 28 werden mittels einer geeigneten Mikro-Materialbearbeitung, vorzugsweise mittels der an sich bekannten Lasertechnologie, beispielsweise mit einem Excimerlaser hergestellt.

An dieser Stelle wird darauf hingewiesen, dass bei der laserstrukturierten Feinbearbeitung der einzelnen Röhrchen 25.1 bis 25.5 an der Oberfläche und/oder an den nicht näher bezeichneten Kanten der einzelnen ersten Ausnehmungen 32,33,36,36' und 38 (Fig.5,5a,6,7) sowie an den einzelnen Ringteilen 29 (Fig.8,8a,9) und an den Kanten des dazwischen angeordneten und mit den zweiten Ausnehmungen 28 versehenen Segmentteils 27 entstandene Grate oder dergleichen mit geeigneten Mitteln entfernbar sind.

Zum Entfernen überstehender Kanten, Auffaserungen oder dergleichen sind verschiedene Feinbearbeitungsverfahren, beispielsweise das thermische Entgratungs-Verfahren (thermal energy machining), Honen, Läppen oder dergleichen bekannt, wobei mittels der einzelnen Verfahren vorhandene Grate auch an unzugänglichen Stellen entfernt werden können. Als besonders vorteilhaft hat sich zudem ergeben, dass sämtliche Kanten, insbesondere aber die Kanten an den einzelnen kreisbogenförmigen Ringteilen 29 (Fig.8) sowie an dem in axialer Richtung orientierten Segmentteil 27 mit einem Radius, beispielsweise mit einem Radius von etwa 0,025 mm bis 0,2 mm abgerundet werden.

Mit den vorstehend erwähnten Feinbearbeitungsverfahren wird das einzelne Röhrchen 25.1 bis 25.5 mit absolut glatt polierter Oberfläche und abgerundeten Kanten hergestellt und somit ein verletzungsfreies Einführen (Fig.3,3a) des Implantats in den Schlemmschen Kanal 15 gewährleistet.

Das Röhrchen 25.1 bis 25.5 ist beispielsweise aus biologisch verträglichem flexiblem Material, beispielsweise aus polymerem Material mit thermisch- oder mechanischem Formgedächtniseffekt hergestellt und beispielsweise bogenförmig biegbar und in Bezug auf den Querschnitt verformbar in das Lumen 16 des Schlemmschen Kanals 15 einführbar und infolge der Körpertemperatur in die vorgegebene ursprüngliche Form zurückführbar. Vorzugweise ist das Röhrchen 25.1 bis 25.5 beispielsweise aus Gold oder Nitinol hergestellt und mit einer Beschichtung, vorzugsweise mit einer Heparin-Beschichtung versehen.

Zum Einführen eines der vorstehend beschriebenen und als Implantat ausgebildeten Röhrchen 25.1 bis 25.5 in das Lumen 16 des Schlemmschen Kanals 15 wird dieser zunächst anhand der an sich bekannten Kanaloplastik-Methode mittels eines flexiblen Mikrokatheters schonend zirkulär dilatiert und gleichzeitig oder anschliessend ein hochmolekulares Viskoelastikum injiziert. Nach erfolgter mechanischer Dilatation wird das Röhrchen, wie beispielsweise in Fig.3 und Fig.3a schematisch dargestellt, in den gedehnten Schlemmschen Kanal 15 eingeführt. Mit dem vorstehend im einzelnen beschriebenen sowie in den einzelnen Figuren dargestellten Röhrchen 25.1 bis 25.5 wird das Lumen 16 des zirkulären Schlemmschen Kanals 15 permanent offen gehalten und der trans-trabekuläre Abfluss des Kammerwassers gewährleistet.

### BEZUGSZEICHEN-LISTE

- 10: Auge
- 11: Hornhaut
- 12: Regenbogenhaut und Teilstück 12'
- 13: Lederhaut (Sklera) mit Skleralappen 13' und Innenseite 13"
- 14: Linse und Pupille 14'
- 15: Schlemmsche Kanal mit Innenwand 15'
- 16: Lumen
- 17: Sklerabett, Auflagefläche 17', Innenfläche 17"
- 18: Trabekulargewebe
- 19: Zonulafasern
- 20: Kammeerwasservenen, Kanälchen 20' und Öffnung 20"
- 21: Pfeilrichtung
- 22: Öffnung und 22'

- 25: Rohling
- 25.1: Röhrchen, zyl. Mantel 26.1 und Teilstücke 26.1.1
- 25.2: Röhrchen, zyl. Mantel 26.2 und Teilstücke 26.2.1
- 25.3: Röhrchen, zyl. Mantel 26.3 und Teilstücke 26.3.1
- 25.4: Röhrchen, zyl. Mantel 26.4 und Teilstücke 26.4.1
- 25.5: Röhrchen, zyl. Mantel 26.5 und Teilstücke 26.5.1
- 26: Zylindrischer Mantel des Rohlings 25
- 27: Segmentteil, kreisbogenförmig
- 28: Zweite Ausnehmungen, Stege 28'
- 29: Ringteile und Teilstücke 29'
- 30: Innenraum
- 31: Wand
- 32: Erste Ausnehmungen (quadratisch, oval oder rund)
- 33: Erste Ausnehmungen (eckiges oder ovales Langloch)
- 34: Steg
- 35: Steg und 35'
- 36: Ausnehmungen und 36'
- 37: Z-förmiger Spalt oder Schlitz
- 38: Abstand der Ringteile
- V: Vorderkammer und Kammerwinkel V'
- H: Hinterkammer
- X: Pfeilrichtung für radiale Relativbewegung der Teilstücke 29'
- Z: Längsachse
- R,R': Reihen der ersten Ausnehmungen 32 und 33
- 1,1': Fliessrichtung des Kammerwassers

## Patentansprüche

1. Implantat zur Behandlung von Glaukom, bestehend aus einem in den Schlemmschen Kanal (15) einführbaren Röhrchen, wobei das Röhrchen flexibel biegsam ausgebildet ist und eine Längsachse (Z) aufweist, und wobei das Röhrchen einen Innenraum (30) und mehrere in der Wand in axialer Richtung im Abstand zueinander angeordnete erste Ausnehmungen (38) aufweist, um über die ersten Ausnehmungen (38) zwischen dem Trabekulargewebe (18), dem Innenraum (30) des Röhrchens sowie dem episkleralen Venensystem (20) eine Verbindung zu bilden, wobei die ersten Ausnehmungen (38) als geometrisches Muster verteilt zueinander in der Wand des Röhrchens (25.4, 25.5) angeordnet sind, und wobei das Röhrchen ein im Profilquerschnitt kreisbogenförmig ausgebildetes und in axialer Richtung orientiertes Segmentteil (27) aufweist, **dadurch gekennzeichnet, dass** die bezüglich der Längsachse (Z) in Umfangsrichtung von der einen Seite bis zu der gegenüberliegenden Seite des Segmentteils (27) orientierten ersten Ausnehmungen (38) einen Öffnungswinkel (W) aufweisen, der in einem Bereich zwischen 290° bis 310° liegt, und dass das Segmentteil (27) mehrere zweite Ausnehmungen (28) aufweist, welche in axialer Richtung des Röhrchens (25.4, 25.5) im Abstand zueinander angeordnet und analog der ersten Ausnehmungen (38) mit dem Innenraum (30) des Röhrchens verbunden sind.

2. Implantat nach Anspruch 1, **dadurch gekennzeichnet, dass** die in dem Segmentteil (27) mittels Stege (28') im Abstand zueinander angeordneten zweiten Ausnehmungen (28) jeweils als rechteckiges Langloch ausgebildet und jeweils in axialer Richtung gleich gross oder unterschiedlich gross ausgebildet sind.

3. Implantat nach Anspruch 2, **dadurch gekennzeichnet, dass** die als rechteckiges Langloch ausgebildeten und in dem Segmentteil (27) angeordneten zweiten Ausnehmungen (28) jeweils an den gegenüberliegenden Enden halbkreisförmig ausgebildet sind.

4. Implantat nach Anspruch 2 oder 3, **dadurch gekennzeichnet, dass** das Röhrchen (25.4, 25.5) mehrere an dem in axialer Richtung orientierten Segmentteil (27) im Abstand (38') zueinander angeordnete und kreisbogenförmig ausgebildete Ringteile (29) und dazwischen angeordnete erste Ausnehmungen (38) aufweist.

5. Implantat nach Anspruch 4, **dadurch gekennzeichnet, dass** die Ringteile (29) in Umfangsrichtung an der dem Segmentteil (27) gegenüberliegenden Seite jeweils durch einen Spalt, beispielsweise durch einen z-förmig ausgebildeten Spalt (37) in zwei radial relativ zueinander spreizbare Teilstücke (29') unterteilt sind.

6. Implantat nach Anspruch 1, **dadurch gekennzeichnet, dass** das Röhrchen (25.4, 25.5) jeweils aus biologisch verträglichen Material, vorzugsweise aus polymerem Material mit thermisch- oder mechanischem Formgedächtniseffekt hergestellt und mit einer Heparin-Beschichtung versehen ist.

7. Implantat nach Anspruch 6, **dadurch gekennzeichnet, dass** das Röhrchen (25.4, 25.5) etwa kreisbogenförmig biegbar und in Bezug auf den Querschnitt verformbar in das Lumen (16) des freigelegten zirkulären Schlemmschen Kanals (15) einführbar und infolge der Körpertemperatur in die vorgegebene ursprüngliche Form zurückführbar ist.

8. Implantat nach Anspruch 1, **dadurch gekennzeichnet, dass** das Röhrchen (25.4, 25.5) aus biologisch verträglichem Material, beispielsweise aus Gold oder Nitinol hergestellt ist.

## Claims

1. Implant for the treatment of glaucoma, consisting of a tube which can be introduced into Schlemm's canal (15), wherein the tube is of flexible design and has a longitudinal axis (Z), and wherein the tube has an interior space (30) and a plurality of first recesses (38) arranged at a distance from one another in the axial direction in the wall, to form a connection via the first recesses (38) between the trabecular tissue (18), the interior (30) of the tube and the episcleral venous system (20), the first recesses (38) being distributed as a geometric pattern with respect to one another in the wall of the tube (25.4, 25.5), and the tube having a segment part (27) which is circularly arc-shaped in the profile cross-section and oriented in the axial direction, **characterized in that** the first recesses (38), which are oriented in the circumferential direction with respect to the longitudinal axis (Z) from one side to the opposite side of the segment part (27), have an opening angle (W) which is in a range between 290° and 310°, and **in that** the segment part (27) has a plurality of second recesses (28) which are arranged in the axial direction of the tube (25.4, 25.5) at a distance from one another, and are connected to the interior (30) of the tube analogously to the first recesses (38).

2. Implant according to claim 1, **characterized in that** the second recesses (28) arranged in the segment part (27) at a distance from one another by means of webs (28') are each formed as a rectangular elongated hole and are each of the same or different size in the axial direction.

3. Implant according to claim 2, **characterized in that** the second recesses (28), which are formed as a rectangular elongated hole and are arranged in the segment part (27), are each formed in a semicircular manner at the opposite ends.

4. Implant according to claim 2 or 3, **characterised in that** the tube (25.4, 25.5) has a plurality of ring parts (29) arranged at a distance (38') from one another on the segment part (27) oriented in the axial direction and of circular arc configuration and first recesses (38) arranged between them.

5. Implant according to Claim 4, **characterized in that** the ring parts (29) are divided in the circumferential direction on the side opposite the segment part (27) in each case by a gap, for example by a z-shaped gap (37), into two sections (29') which can be spread radially relative to one another.

6. Implant according to claim 1, **characterized in that** the tube (25.4, 25.5) is made of biologically compatible material, preferably polymeric material with a thermal or mechanical shape memory effect, and is provided with a heparin coating.

7. Implant according to Claim 6, **characterized in that** the tube (25.4, 25.5) can be bent approximately in the shape of a circular arc and can be introduced into the lumen (16) of the exposed circular Schlemm's canal (15) so as to be deformable with respect to the cross-section and can be returned to the predetermined original shape as a result of the body temperature.

8. Implant according to claim 1, **characterized in that** the tube (25.4, 25.5) is made of biologically compatible material, for example gold or nitinol.

## Revendications

1. Implant pour le traitement du glaucome, constitué d'un tube qui peut être introduit dans le canal de Schlemm (15), le tube est flexible et présente un axe longitudinal (Z), et le tube présente un espace intérieur (30) et plusieurs premiers évidements (38) disposés à distance les uns des autres en direction axiale dans la paroi, pour former une liaison par les premiers évidements (38) entre le tissu trabéculaire (18), l'intérieur (30) du tube et le système veineux épiscléral (20), les premiers évidements (38) étant répartis en un motif géométrique les uns par rapport aux autres sur la paroi du tube (25.4, 25.5), et le tube comportant une partie de segment (27) en forme d'arc de cercle dans la section transversale du profilé et orientée dans la direction axiale, **caractérisé en ce que** les premiers évidements (38) qui sont orientés dans la direction périphérique par rapport à l'axe longitudinal (Z) d'un côté vers le côté opposé de la partie de segment (27) présentent un angle d'ouverture (W) compris entre 290° et 310°, et que la partie de segment (27) présente plusieurs seconds évidements (28) disposés dans la direction axiale du tube (25.4, 25.5) à distance l'un de l'autre et sont reliés à l'intérieur (30) du tube de manière analogue aux premiers évidements (38).

2. Implant selon la revendication 1, **caractérisé en ce que** les deuxièmes évidements (28) disposés à distance l'un de l'autre dans la partie de segment (27) au moyen de nervures (28') sont chacun formés comme un trou oblong rectangulaire et ont chacun une taille identique ou différente dans la direction axiale.

3. Implant selon la revendication 2, **caractérisé en ce que** les deuxièmes évidements (28), qui sont formés sous la forme d'un trou oblong rectangulaire et qui sont disposés dans la partie de segment (27), sont formés chacun en demi-cercle aux extrémités opposées.

4. Implant selon la revendication 2 ou 3, **caractérisé en ce que** le tube (25.4, 25.5) comporte plusieurs parties annulaires (29) disposées à une distance (38') les unes des autres sur la partie de segment (27) orientée dans la direction axiale et présentant une configuration en arc de cercle et des premiers évidements (38) disposés entre elles.

5. Implant selon la revendication 4, **caractérisé en ce que** les parties annulaires (29) sont divisées dans la direction circonférentielle du côté opposé à la partie de segment (27) par un espace, par exemple par un espace en Z (37), en deux parties (29') qui peuvent être réparties radialement l'une par rapport à l'autre.

6. Implant selon la revendication 1, **caractérisé en ce que** le tube (25.4, 25.5) est réalisé en matériau biologiquement compatible, de préférence en matériau polymère à effet de mémoire de forme thermique ou mécanique, et est pourvu d'un revêtement d'héparine.

7. Implant selon la revendication 6, **caractérisé en ce que** le tube (25.4, 25.5) peut être courbé approximativement en forme d'arc de cercle et peut être introduit dans la lumière (16) du canal de Schlemm circulaire exposé (15) de manière à être déformable par rapport à la section transversale et peut être ramené à sa forme originale prédéterminée en raison de la température du corps.

8. Implant selon la revendication 1, **caractérisé en ce que** le tube (25.4, 25.5) est en matériau biologiquement compatible, par exemple en or ou en nitinol.
